# EUROPEAN PATENT SPECIFICATION

(11) **EP 0 273 310 B1**
(45) Date of publication and mention of the grant of the patent: **21.06.1995**
(21) Application number: 87118728.2
(22) Date of filing: 17.12.1987
(51) Int. Cl.: A61K 31/41, A61K 31/44, A61K 31/47

(54) **5-Aryl-3H-1,2,4-triazol-3-ones and their use as anticonvulsants**
5-Aryl-3H-1,2,4-triazol-3-one und ihre Verwendung als Antikonvulsiva
5-Aryl-3H-1,2,4-triazol-3-ones et leur emploi comme anticonvulsifs

(30) Priority: 19.12.1986 US 944634; 27.08.1987 US 90310
(43) Date of publication of application: 06.07.1988
(73) Proprietor: MERRELL PHARMACEUTICALS INC., Cincinnati Ohio 45215 (US)
(72) Inventor: Miller, Francis P., Loveland Ohio 45140 (US); Kane, John M., Cincinnati Ohio 45236 (US)
(74) Representative: Sgarbi, Renato

(56) References cited:
- EP-A- 0 221 485
- BE-A- 621 842
- DD-A- 153 953
- DD-A- 160 447
- DE-A- 1 126 882
- US-A- 3 514 466
- JOURNAL OF MEDICINAL CHEMISTRY, vol. 14, no. 3, 1971, pages 260-262; M.Y. MHASALKAR et al.: "Further studies in substituted 4H-1,2,4-triazoles for possible hypoglycemic activity"
- SYNTHESIS, vol. 10, October 1987, pages 912-914; J.M. KANE: "The bis(tricyclohexylstannyl) sulfide thionation of 3H-1,2,4-triazol-3-ones"
- JOURNAL OF PHARM. SCIENCES, vol. 66, no. 7, July 1977, pages 971-975; S.S. PARMAR et al.: "Anticonvulsant activity and selective inhibition of NAD-dependent oxidations in rat brain homogenates by newer mercaptotriazoles"
- RESEARCH COMMUNICATIONS IN CHEMICAL PATHOLOGY AND PHARMACOLOGY, vol. 37, no. 3, September 1982, pages 499-502; R.K. JAISWAL et al.: "Anticonvulsant activity and monoamine oxidase inhititory properties of substituted 1,2,4-triazoles"

## Description

This invention relates to the use of 5-aryl-3H-1,2,4-triazol-3-ones for preparing a medicament for treatment of seizure disorders.

M.Y. Mhasalkar, et al., (J. Med. Chem. 14, 260-262, 1971) teach that a compound of formula I has hypoglycemic activity. U.S.-A-3,514,466; BE-A-621,842 and DE-A-1,126,882 teach that various compounds of formula I have sedative and hypnotic activity. ZA 65/1537 and NL 6504121 teach that some compounds of formula I have anti-inflammatory, antipyretic and analgetic activity. DD-A-160,447; DD-A-153,953 and BE-A-894,856 teach that some compounds of formula I are herbicides. U.S.-A-4,414,221 teaches that some compounds of formula I are insecticides and acaricides. JP 50-63119 teaches that some compounds of formula I have anticoccidial activity.

More specifically this invention relates to the use of a compound of the formula: 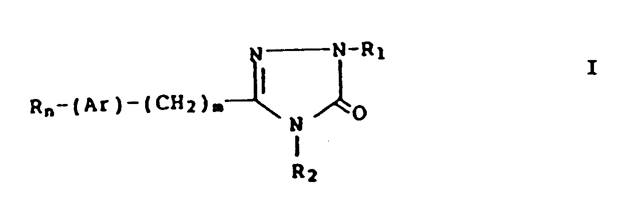
wherein
Ar represents phenyl, naphthyl or an aromatic heterocyclic moiety, selected from 2-, 3- or 4-pyridyl, 2- or 3-furyl, 2- or 3-thienyl, 2- or 3-pyrrolyl, N-(C₁₋₆ alkyl)-pyrrolyl, 6-isoquinolyl, 6-quinolyl and 3-quinolyl,
R₁ is hydrogen or C₁₋₆ alkyl,
R₂ is C₁₋₆ alkyl,
R is C₁₋₆ alkyl, C₁₋₆ alkoxy, hydroxy, halogeno, or trifluoromethyl, and
n is zero, 1 or 2, or
Rₙ-(Ar) is methylenedioxyphenyl, and
m is zero, 1 or 2,
and the pharmaceutically acceptable salts of the compounds wherein Ar is a nitrogen-containing heterocyclic moiety for preparing a medicament for treating seizure disorders.

For R, preferably halogeno represents chloro or fluoro, and methyl and ethyl represent the preferred C₁-C₆ alkyl moieties, although all the straight, branched and cyclic manifestations thereof such as n-propyl, cyclopentyl, cyclohexyl and cyclopropyl are herein included. C₁₋C₆ alkoxy radicals include ethers having alkyl moieties paralleling the C₁₋₆ alkyl group.

R₁ and R₂ are preferably methyl or ethyl, although any straight or branched C₁₋₆ alkyl group may be used.

When "Ar" is phenyl, preferably n is one, representing a mono-substituted phenyl moiety with the R-substitutent being a group located at any of the ortho, meta or para positions, although the ortho- and para-substituted compounds are preferred. When Ar is disubstituted (i.e., n is 2), the 2,3-; 2,4-; 2,5-; 2,6-; 3,4-; and 3,5- positions are contemplated. The tautomeric forms are included for each of the compounds embraced within formula I. Except when Ar is phenyl,it is preferred that m is zero. When Ar is phenyl it is preferred that m is zero or one.

When "Ar" of formula I represents a heterocyclic moiety such heterocyclic moieties as 2-, 3- or 4-pyridyl, 2- or 3-furyl, 2- or 3-thienyl, 2- or 3-pyrrolyl, N-(C₁₋₆ alkyl)-pyrrolyl, 6-isoquinolyl, 6-quinolyl and 3-quinolyl are contemplated. Preferred is 4-pyridyl, with or without an R substituent. State of the art salts formed with the heterocyclic moieties are generally employed, with the hydrochloride being one of convenience and general applicability: such salts being formed by standard techniques well known in the art.

When "Ar" represents naphthyl, the preferred isomer is 2-naphthyl with the R moiety being attached thereto at any of the available positions, although positions 5-, 6-, 7- or 8- are preferred for either the mono- or di-R-substituted naphthyl compounds of formula I.

Another object of the present invention is represented by a compound of formula I wherein:
- Ar: represents phenyl, naphthyl, or an aromatic heterocyclic moiety selected from 2-, 3- or 4-pyridyl, 2- or 3-furyl, 2- or 3-thienyl, 2- or 3-pyrrolyl, N-(C₁₋₆ alkyl)-pyrrolyl, 6-isoquinolyl, 6-quinolyl and 3-quinolyl,
- R₁: is hydrogen or C₁₋₆ alkyl,
- R₂: is C₁₋₆ alkyl,
- R: is C₁₋₆ alkyl, C₁₋₆ alkoxy, hydroxy, halogeno, or trifluoromethyl, and
- n: is 1 or 2, or
- Rₙ-(Ar): is methylenedioxyphenyl, and
- m: is zero, 1 or 2,
with the proviso that when Rₙ-(Ar)-(CH₂)ₘ-represents 4-chlorophenyl and R₂ represents ethyl, R₁ cannot represent hydrogen, and when Rₙ-(Ar)-(CH₂)ₘ-represents phenyl and R₁ represents methyl, R₂ cannot represent methyl or ethyl,
and the pharmaceutically acceptable salts of the compounds wherein Ar is a nitrogen-containing heterocyclic moiety for use as a medicament.

A further object of the present invention is represented by the compounds of formula I wherein Rₙ-(Ar)-(CH₂)ₘ- represents 4-chlorophenyl, R₁ represents methyl and R₂ represents ethyl or wherein Rₙ-(Ar)-(CH₂)ₘ- represents 2-chlorophenyl, R₁ represents hydrogen and R₂ represents methyl.

The compounds of Formula I may readily be prepared using processes and techniques analogously known in the art, for example in the method of S. Kuboda and M. Uda, Chem. Pharm. Bull. 21, 1342 (1979), as seen by the following reaction scheme: 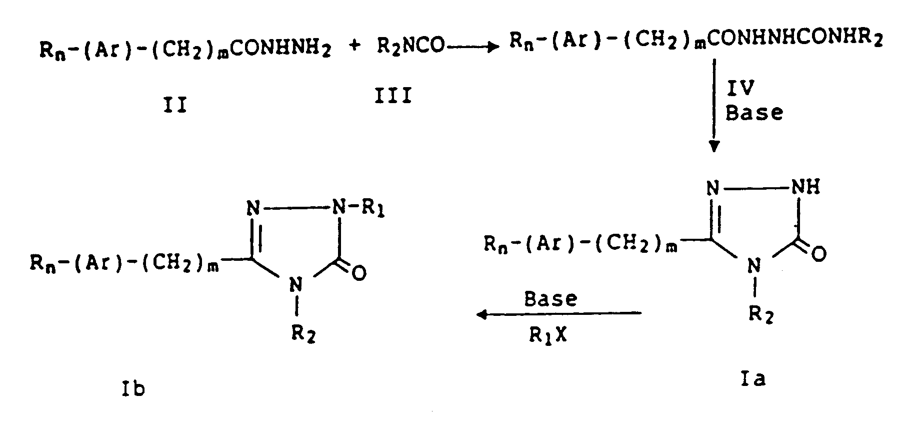
wherein Rₙ-(Ar)-(CH₂)ₘ-, R₁ and R₂ are as defined in formula I, and X is a suitable leaving group.

The preparation of the 1-aroylsemicarbazides (IV) is readily effected by reacting an aroyl hydrazide (II) with an R₂-substituted isocyanate (III) by contacting the reactants together in a suitable aprotic solvent, preferably one in which the hydrazide reactant is soluble, e.g., tetranydrofuran (THF),CHCl₃, CH₂Cl₂, benzene, toluene, Et₂O and the like. The reaction is quite rapid and may be carried out at 0°C to about room temperature and, although the reaction proceeds rapidly, the mixture may be left for 24 hours without any significant decrease in yield. The required hydrazides and isocyanates are readily available but may be prepared by known techniques quite obvious to one of ordinary skill in the art.

The desired 5-aryl-2,4-dihydro-3H-1,2,4-triazol-3-ones (Ia) may be prepared by reacting the semicarbazides (IV) with a base, preferably an aqueous alkali metal hydroxide (e.g., NaOH, KOH) at about 50-120°C, although reflux temperatures are preferred. Normal reaction time is about 7 hours, although 4-24 hours may be needed depending on the temperature of the mixture.

The desired 2,4-disubstituted-2,4-dihydro-3H-1,2,4-triazol-3-ones (Ib) may be prepared by reacting the 4-substituted-2,4-dihydro-3H-1,2,4-triazol-3-ones (Ia) with an appropriate R₁X reactant wherein X is a suitable leaving group, e.g., Cl, Br, OSO₂CF₃ and the like. Preferably the reaction takes place in a solution of an aqueous alkali metal hydroxide, (e.g., KOH, NaOH) although more reactive bases (e.g., NaH, KH, LDA) may be used if the reaction is effected under aprotic dry conditions. The reaction preferably takes place at room temperature over periods of about 18 hours to two weeks.

The following specific examples are given to illustrate the preparation of the compounds

### Preparation of Intermediate 1-Aroyl-4-substituted semicarbazides

### EXAMPLE 1

### 1-(4-Chlorobenzoyl)-4-ethylsemicarbazide

A stirred suspension of 4-chlorobenzoic acid, hydrazide (17.1 g, 1.00 x 10⁻¹ mole), and THF (425 ml) was warmed until homogeneous, at which time ethyl isocyanate (8.7 ml, 1.1 x 10⁻¹ mole) was added via syringe. A precipitate soon formed. After stirring overnight the reaction was diluted with Et₂O and the precipitate was collected by filtration affording a colorless powder: 23.7 g (98%). Crystallization from ethanol gave a colorless solid: 21.4 g (88%), mp 237-239°.

### EXAMPLE 2

### 1-(4-Pyridoyl)-4-methylsemicarbazide

When, in the procedure of Example 1, isonicotinic acid is substituted for 4-chlorobenzoic acid, the title compound is obtained.

### Preparation of 5-Aryl-4-substituted-2,4-dihydro-3H-1,2,4-triazol-3-ones

### EXAMPLE 3

### 5-(4-Chlorophenyl)-2,4-dihydro-4-ethyl-3H-1,2,4-triazol-3-one

1-(4-chlorobenzoyl)-4-ethylsemicarbazide (23.7 g, 9.81 x 10⁻² mole) and 1 molar aqueous NaOH (118 ml, 1.18 x 10⁻¹ mole) were stirred and warmed to reflux. After refluxing 23 hours, heating was discontinued and the reaction was acidified by the dropwise addition of 1 molar aqueous hydrochloric acid (130 ml, 1.30 x 10⁻¹ mole). A colorless solid formed as the reaction was acidified and, after cooling in an ice bath, this was collected by filtration. Crystallization from isopropanol gave colorless spars: 18.2 g (83%), mp 188-189°.

### EXAMPLE 4

### 2,4-Dihydro-4-methyl-5-(4-pyridinyl)-3H-1,2,4-triazol-3-one

When, in the procedure of Example 3, 1-(4-pyridoyl)-4-methylsemicarbazide is substituted for 1-(4-chlorobenzoyl)-4-ethylsemicarbazide, the title compound is obtained. Mp 249-251°C.

### Preparation of 5-Aryl-2,4-dihydro-2,4-disubstituted-3H-1,2,4-triazol-3-ones

### EXAMPLE 5

### 5-(4-Chlorophenyl)-2,4-dihydro-4-ethyl-2-methyl-3H-1,2,4-triazol-3-one

To a stirred, room temperature solution of 5-(4-chlorophenyl)-2,4-dihydro-4-ethyl-3H-1,2,4-triazol-3-one (6.00 g, 2.68 x 10⁻² mole) and 1 molar aqueous NaOH (30.0 ml, 3.00 x 10⁻² mole) was added a solution of methyl iodide (2.5 ml, 4.0 x 10⁻² mole) and ethanol (10 ml). After stirring overnight at room temperature, the reaction mixture was transferred to a separatory funnel where it was extracted three times with EtOAc. The EtOAc extracts were combined, washed with saturated aqueous NaCl, and dried over Na₂SO₄. The drying agent was removed by filtration and the filtrate was evaporated at reduced pressure leaving an oil which slowly solidified. Chromatography and crystallization from cyclohexane gave small colorless needles: 3.4 g (53%), mp 73-75°.

In a similar manner the following compounds also may be prepared.

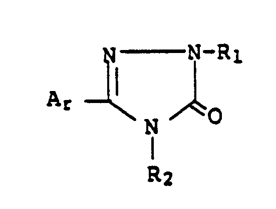

| Aᵣ | R₁ | R₂ | mp (°C) |
|---|---|---|---|
| C₆H₅ | H | CH₃ | 177-178 |
| C₆H₅ | CH₃ | CH₃ | 140-141 |
| C₆H₅ | C₂H₅ | CH₃ | 87-89 |
| C₆H₅ | H | C₂H₅ | 163-165 |
| C₆H₅ | CH₃ | C₂H₅ | oil |
| 2-ClC₆H₄ | H | CH₃ | 168-170 |
| 2-ClC₆H₄ | CH₃ | CH₃ | 61-63 |
| 4-ClC₆H₄ | H | CH₃ | 213-215 |
| 4-ClC₆H₄ | CH₃ | CH₃ | 126-128 |
| 4-ClC₆H₄ | C₂H₅ | CH₃ | 79-81 |
| 4-ClC₆H₄ | H | C₂H₅ | 188-189 |
| 4-ClC₆H₄ | CH₃ | C₂H₅ | 73-75 |
| 4-ClC₆H₄ | C₂H₅ | C₂H₅ | 62-64 |
| 4-ClC₆H₄ | n-C₃H₇ | C₂H₅ | oil |
| 2-FC₆H₄ | H | CH₃ | 189-191 |
| 2-FC₆H₄ | CH₃ | CH₃ | 69-71 |
| 4-FC₆H₄ | H | CH₃ | 216-218 |
| 4-FC₆H₄ | CH₃ | CH₃ | 104-106 |
| 3,4-Cl₂C₆H₃ | H | CH₃ | 170-172 |
| 3,4-Cl₂C₆H₃ | CH₃ | CH₃ | 107-109 |
| 4-CH₃C₆H₄ | H | CH₃ | 206-208 |
| 4-CH₃C₆H₄ | CH₃ | CH₃ | 92-94 |
| 4-CH₃O-3-(n-C₄H₉O)C₆H₃ | H | CH₃ | 96-98 |
| 4-CH₃O-3-(n-C₄H₉O)C₆H₃ | CH₃ | CH₃ | 112-114 |
| 4-CH₃O-3-(cyclo-C₅H₉O)C₆H₃ | H | CH₃ | 184-186 |
| 4-CH₃O-3-(cyclo-C₅H₉O)C₆H₃ | CH₃ | CH₃ | 153-155 |

The pharmacological profile of these compounds and their relative potencies may readily be demonstrated through standard laboratory tests indicative of compounds known to be useful as anticonvulsants suitable for use in the treatment of seizure disorders, particularly idiopathic epilepsy.

For example, in the evaluation and characterization of the anticonvulsant and GABAergic activity and to observe a suitable pharmacological profile of the compounds of this invention, it is convenient to employ such tests as the antagonism of 3-mercaptopropionic acid-induced convulsions, an assay performed on mice wherein wild running fits or generalized seizures are induced by 3-mercaptopropionic acid; the antagonism of strychnine-induced seizures in mice; the antagonism to maximal electroshock, an assay performed in mice wherein seizures are caused by the administration of electroshock; and the antagonism to pentylenetetrazol, an assay to measure the prevention of seizures caused by administration of pentylenetetrazol.

Compounds which inhibit pentylenetetrazol-induced seizures in mice are known to possess anticonvulsant and anti-anxiety effects. An appropriate dose of test compound is administered to groups of mice and, at a selected time thereafter, pentylenetetrazol, prepared as a solution in distilled water such that 10 ml/kg delivers a dose of 60 mg/kg, is administered by rapid intravenous injection. Absence of clonic convulsions for 2 minutes after pentylenetetrazol is considered significant protection. Prevention of tonic extensor convulsions is also reported and usually occurs at a dose lower than that required to block clonic convulsions. Inhibition of clonic seizures induced by this dose of pentylenetetrazol is evidence of potential anticonvulsant/anti-anxiety activity. Against seizures caused by pentylenetetrazol, 5-(4-chlorophenyl)-2,4-dihydro-4-ethyl-2-methyl-3H-1,2,4-triazol-3-one has an ED₅₀ of 16.5 mg/kg.

Based upon standard laboratory methodology (including comparative tests with known anticonvulsants), the compounds of this invention will exert anticonvulsant activity useful in the treatment of idiopathic epilepsy at oral dosage levels of about 0.25 to 25 mg/kg of body weight per day. Of course the degree of severity of the disease, age of the patient and other factors normally considered by the attending diagnostician will influence the individual regimen for each patient. In general, the parenterally administered doses are about 1/4 to 1/2 that of the orally administered dose.

For oral administration the compounds can be formulated into solid or liquid preparations such as capsules, pills, tablets, troches, powders, solutions, suspensions or emulsions. The solid unit dosage forms can be a capsule which can be of the ordinary gelatin type containing, for example, lubricants and inert fillers such as lactose, sucrose or cornstarch. In another embodiment the compounds of general formula I can be tableted with conventional tablet bases such as lactose, sucrose and cornstarch, in combination with binders such as acacia, cornstarch or gelatin, disintegrating agents such as potato starch or alginic acid, and a lubricant such as stearic acid or magnesium stearate.

For parenteral administration, the compounds may be administered as injectable dosages of a solution or suspension of the compound in a physiologically acceptable diluent with a pharmaceutical carrier which can be a sterile liquid such as water, alcohol, oils and other acceptable organic solvents, with or without the addition of a surfactant and other pharmaceutically acceptable adjuvants. Illustrative of oils which can be employed in these preparations are those of petroleum, animal, vegetable or synthetic origin, for example, peanut oil, soybean oil and mineral oil. In general, water, saline, aqueous dextrose and related sugar solutions, ethanol, glycols such as propylene glycol or polyethylene glycol, or 2-pyrrolidone are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic®, a silicone rubber manufactured by the Dow-Corning Corporation.

As is true for most classes of compounds generally suitable as therapeutic agents, certain subgeneric groups and specific members of that class, in the light of their overall biological profile, are preferred. In this instance those compounds wherein m is zero are preferred, with those wherein m is one being next preferred. The preferred Ar moiety is phenyl. The preferred R substituent is chloro, with the chloro being at the 2- or 4-positions of the aromatic moiety being preferred. It is preferred to have an alkyl substituent at both of the R₁ and R₂ positions with methyl and ethyl being the preferred groups. Particularly preferred compounds are
5-(4-chlorophenyl)-2,4-dihydro-4-ethyl-2-methyl-3H-1,2,4-triazol-3-one
5-(4-chlorophenyl)-2,4-diethyl-2,4-dihydro-3H-1,2,4-triazol-3-one
5-(2-chlorophenyl)-2,4-dihydro-4-methyl-3H-1,2,4-triazol-3-one
5-(2-chlorophenyl)-2,4-dihydro-2,4-dimethyl-3H-1,2,4-triazol-3-one.

In the present description and claims the term therapeutic "treatment" includes any kind of treatment such as prophylaxis and cure.

## Claims (Claims for the following Contracting State(s): AT, BE, CH, DE, FR, GB, GR, IT, LI, LU, NL, SE)

1. Use of a compound of the formula 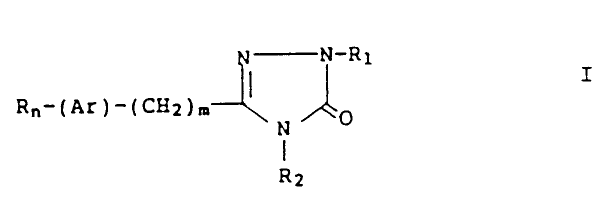 wherein
Ar represents phenyl, naphthyl, or an aromatic heterocyclic moiety selected from 2-, 3- or 4-pyridyl, 2- or 3-furyl, 2- or 3-thienyl, 2- or 3-pyrrolyl, N-(C₁₋₆ alkyl)-pyrrolyl, 6-isoquinolyl, 6-quinolyl and 3-quinolyl,
R₁ is hydrogen or C₁₋₆ alkyl,
R₂ is C₁₋₆ alkyl,
R is C₁₋₆ alkyl, C₁₋₆ alkoxy, hydroxy, halogeno, or trifluoromethyl, and
n is zero, 1 or 2, or
Rₙ-(Ar) is methylenedioxyphenyl, and
m is zero, 1 or 2,
and the pharmaceutically acceptable salts of the compounds wherein Ar is a nitrogen-containing heterocyclic moiety, for preparing a medicament for treating seizure disorders.

2. Use of a compound of claim 1 wherein Ar in said compound represents phenyl.

3. Use of a compound of claim 1 or 2 wherein m in said compound represents zero.

4. Use of a compound of claim 1, 2 or 3 wherein R₁ in said compound represents C₁₋₆ alkyl.

5. Use of a compound of claim 1, 2 or 3 wherein R₁ in said compound represents methyl or ethyl.

6. Use of a compound of claim 1 wherein Ar in said compound is phenyl, m is zero and R is halogeno.

7. Use of a compound of claim 6 wherein R in said compound is chloro.

8. Use of a compound of claim 1 wherein Rₙ-(Ar)-(CH₂)ₘ-in said compound represents 4-chlorophenyl, R₁ is methyl and R₂ is ethyl.

9. Use of a compound of claim 1 wherein Rₙ-(Ar)-(CH₂)ₘ-in said compound represents 4-chlorophenyl, R₁ is hydrogen and R₂ is ethyl.

10. Use of a compound of claim 1 wherein Rₙ-(Ar)-(CH₂)ₘ-in said compound represents 2-chlorophenyl, R₁ is hydrogen and R₂ is methyl.

11. A compound of the formula 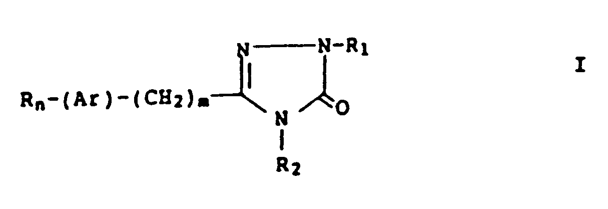 wherein
Ar represents phenyl, naphthyl, or an aromatic heterocyclic moiety selected from 2-, 3- or 4-pyridyl, 2- or 3-furyl, 2- or 3-thienyl, 2- or 3-pyrrolyl, N-(C₁₋₆ alkyl)-pyrrolyl, 6-isoquinolyl, 6-quinolyl and 3-quinolyl,
R₁ is hydrogen or C₁₋₆ alkyl,
R₂ is C₁₋₆ alkyl,
R is C₁₋₆ alkyl, C₁₋₆ alkoxy, hydroxy, halogeno, or trifluoromethyl, and
n is 1 or 2, or
Rₙ-(Ar) is methylenedioxyphenyl, and
m is zero, 1 or 2,
with the proviso that when Rₙ-(Ar)-(CH₂)ₘ-represents 4-chlorophenyl and R₂ represents ethyl, R₁ cannot represent hydrogen, and when Rₙ-(Ar)-(CH₂)ₘ-represents phenyl and R₁ represents methyl, R₂ cannot represent methyl or ethyl,
and the pharmaceutically acceptable salts of the compounds wherein Ar is a nitrogen-containing heterocyclic moiety for use as a medicament.

12. A compound of claim 11 wherein Ar is phenyl.

13. A compound of claim 11 or 12 wherein m is zero.

14. A compound of claim 11, 12, or 13 wherein R₂ is methyl or ethyl.

15. A compound of claim 11 wherein Ar is phenyl, m is zero and R is halogeno.

16. A compound of claim 15 wherein R is chloro.

17. A compound of claim 11 wherein Rₙ-(Ar)-(CH₂)ₘ-represents 2-chlorophenyl, R₁ is hydrogen and R₂ is methyl.

18. A compound of claim 11 wherein Rₙ-(Ar)-(CH₂)ₘ-represents 4-chlorophenyl, R₁ is methyl and R₂ is ethyl.

19. A compound of the formula I 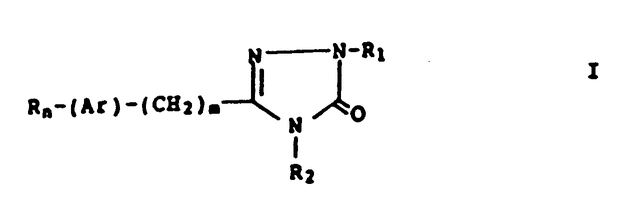 wherein Rₙ-(Ar)-(CH₂)ₘ-represents 4-chlorophenyl, R₁ is methyl and R₂ is ethyl.

20. A compound of the formula I 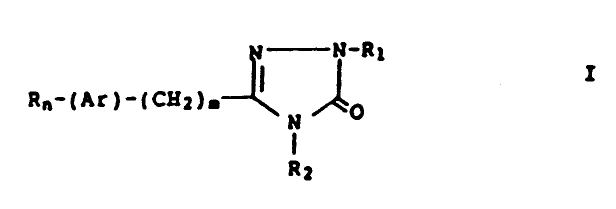 wherein Rₙ-(Ar)-(CH₂)ₘ-represents 2-chlorophenyl, R₁ is hydrogen and R₂ is methyl.

21. A pharmaceutical formulation containing a compound of any one of claims 11 to 20 in admixture with a pharmaceutically acceptable carrier.

22. A process for preparing a compound of the formula 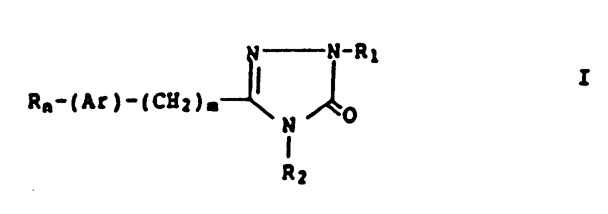 wherein
Rₙ-(Ar)-(CH₂)ₘ- represents 4-chlorophenyl, R₁ represents methyl and R₂ represents ethyl, which comprises reacting a semicarbazide of formula IV
Rₙ-(Ar)-(CH₂)ₘCONHNHCONHR₂
wherein Rₙ-(Ar)-(CH₂)ₘ-is as defined above, with a base at about 50°C-120°C; and transforming the obtained intermediate wherein R₁ represents hydrogen into the compound of formula I by reacting it with a compound of formula R₁X wherein X is a good leaving group and R₁ is methyl.

23. A process for preparing a compound of the formula 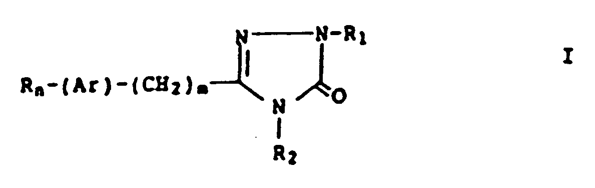 wherein
Rₙ-(Ar)-(CH₂)ₘ- represents 2-chlorophenyl, R₁ represents hydrogen and R₂ represents methyl, which comprises reacting a semicarbazide of formula IV
Rₙ-(Ar)-(CH₂)ₘCONHNHCONHR₂
wherein Rₙ-(Ar)-(CH₂)ₘ-is as defined above, with a base at about 50°C-120°C.

## Claims (Claims for the following Contracting State(s): ES)

1. A process for preparing a compound of the formula 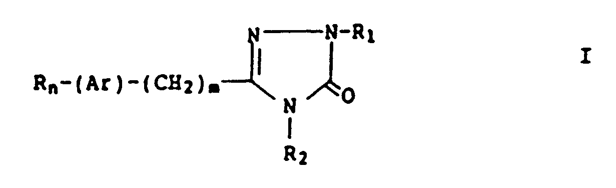 wherein
Rₙ-(Ar)-(CH₂)ₘ- represents 4-chlorophenyl, R₁ represents methyl and R₂ represents ethyl, which comprises reacting a semicarbazide of formula IV
Rₙ-(Ar)-(CH₂)ₘCONHNHCONHR₂
wherein Rₙ-(Ar)-(CH₂)ₘ-is as defined above, with a base at about 50°C-120°C; and transforming the obtained intermediate wherein R₁ represents hydrogen into the compound of formula I by reacting it with a compound of formula R₁X wherein X is a good leaving group and R₁ is methyl.

2. A process for preparing a compound of the formula 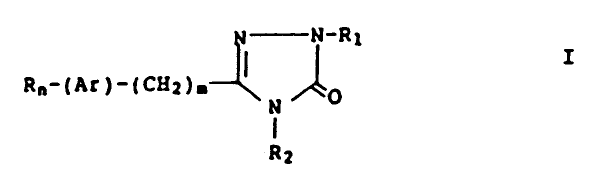 wherein
Rₙ-(Ar)-(CH₂)ₘ- represents 2-chlorophenyl, R₁ represents hydrogen and R₂ represents methyl, which comprises reacting a semicarbazide of formula IV
Rₙ-(Ar)-(CH₂)ₘCONHNHCONHR₂
wherein Rₙ-(Ar)-(CH₂)ₘ-is as defined above, with a base at about 50°C-120°C.

## Patentansprüche (Patentansprüche für folgende(n) Vertragsstaat(en): AT, BE, CH, DE, FR, GB, GR, IT, LI, LU, NL, SE)

1. Verwendung einer Verbindung der Formel 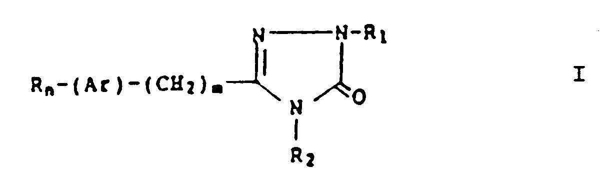 in der
Ar eine Phenyl-, Naphthylgruppe oder eine aromatische, heterocyclische Einheit bedeutet, ausgewählt aus einer 2-, 3- oder 4-Pyridyl-, 2- oder 3-Furyl-, 2- oder 3-Thienyl-, 2- oder 3-Pyrrolylgruppe, einem N-(C₁₋₆-Alkyl)pyrrolylrest, einer 6-Isochinolyl-, 6-Chinolyl- und 3-Chinolylgruppe,
R₁ ein Wasserstoffatom oder ein C₁₋₆-Alkylrest ist,
R₂ ein C₁₋₆-Alkylrest ist,
R ein C₁₋₆-Alkyl-, C₁₋₆-Alkoxyrest, eine Hydroxylgruppe, ein Halogenatom oder eine Trifluormethylgruppe ist, und
n null, 1 oder 2 ist, oder
Rₙ-(Ar) eine Methylendioxyphenylgruppe ist, und
m null, 1 oder 2 ist,
und der pharmazeutisch verträglichen Salze der Verbindungen, in denen Ar eine Stickstoff-enthaltende, heterocyclische Einheit ist, zur Herstellung eines Arzneimittels zur Behandlung von Anfallserkrankungen.

2. Verwendung einer Verbindung nach Anspruch 1, wobei Ar in der Verbindung eine Phenylgruppe bedeutet.

3. Verwendung einer Verbindung nach Anspruch 1 oder 2, wobei m in der Verbindung null ist.

4. Verwendung einer Verbindung nach Anspruch 1, 2 oder 3, wobei R₁ in der Verbindung einen C₁₋₆-Alkylrest bedeutet.

5. Verwendung einer Verbindung nach Anspruch 1, 2 oder 3, wobei R₁ in der Verbindung eine Methyl- oder Ethylgruppe bedeutet.

6. Verwendung einer Verbindung nach Anspruch 1, wobei Ar in der Verbindung eine Phenylgruppe ist, m null ist, und R ein Halogenatom ist.

7. Verwendung einer Verbindung nach Anspruch 6, wobei R in der Verbindung ein Chloratom ist.

8. Verwendung einer Verbindung nach Anspruch 1, wobei Rₙ-(Ar)-(CH₂)ₘ- in der Verbindung eine 4-Chlorphenylgruppe bedeutet, R₁ eine Methylgruppe ist, und R₂ eine Ethylgruppe ist.

9. Verwendung einer Verbindung nach Anspruch 1, wobei Rₙ-(Ar)-(CH₂)ₘ- in der Verbindung eine 4-Chlorphenylgruppe bedeutet, R₁ ein Wasserstoffatom ist, und R₂ eine Ethylgruppe ist.

10. Verwendung einer Verbindung nach Anspruch 1, wobei Rₙ-(Ar)-(CH₂)ₘ- in der Verbindung eine 2-Chlorphenylgruppe bedeutet, R₁ ein Wasserstoffatom ist, und R₂ eine Methylgruppe ist.

11. Verbindung der Formel 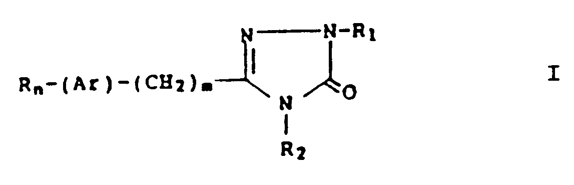 in der
Ar eine Phenyl-, Naphthylgruppe oder eine aromatische, heterocyclische Einheit bedeutet, ausgewählt aus einer 2-, 3- oder 4-Pyridyl-, 2- oder 3-Furyl-, 2- oder 3-Thienyl-, 2- oder 3-Pyrrolylgruppe, einem N-(C₁₋₆-Alkyl)pyrrolylrest, einer 6-Isochinolyl-, 6-Chinolyl- und 3-Chinolylgruppe,
R₁ ein Wasserstoffatom oder ein C₁₋₆-Alkylrest ist,
R₂ ein C₁₋₆-Alkylrest ist,
R ein C₁₋₆-Alkyl-, C₁₋₆-Alkoxyrest, eine Hydroxylgruppe, ein Halogenatom oder eine Trifluormethylgruppe ist, und
n 1 oder 2 ist, oder
Rₙ-(Ar) eine Methylendioxyphenylgruppe ist, und
m null, 1 oder 2 ist,
mit der Maßgabe, daß R₁ kein Wasserstoffatom bedeuten kann, wenn Rₙ-(Ar)-(CH₂)ₘ- eine 4-Chlorphenylgruppe bedeutet und R₂ eine Ethylgruppe bedeutet, und R₂ keine Methyl- oder Ethylgruppe bedeuten kann, wenn Rₙ-(Ar)-(CH₂)ₘ- eine Phenylgruppe bedeutet und R₁ eine Methylgruppe bedeutet,
und die pharmazeutisch verträglichen Salze der Verbindungen, in denen Ar eine Stickstoff-enthaltende, heterocyclische Einheit ist, zur Verwendung als Arzneimittel.

12. Verbindung nach Anspruch 11, wobei Ar eine Phenylgruppe ist.

13. Verbindung nach Anspruch 11 oder 12, wobei m null ist.

14. Verbindung nach Anspruch 11, 12 oder 13, wobei R₂ eine Methyl- oder Ethylgruppe ist.

15. Verbindung nach Anspruch 11, wobei Ar eine Phenylgruppe ist, m null ist, und R ein Halogenatom ist.

16. Verbindung nach Anspruch 15, wobei R ein Chloratom ist.

17. Verbindung nach Anspruch 11, wobei Rₙ-(Ar)-(CH₂)ₘ- eine 2-Chlorphenylgruppe bedeutet, R₁ ein Wasserstoffatom ist, und R₂ eine Methylgruppe ist.

18. Verbindung nach Anspruch 11, wobei Rₙ-(Ar)-(CH₂)m- eine 4-Chlorphenylgruppe bedeutet, R₁ eine Methylgruppe ist, und R₂ eine Ethylgruppe ist.

19. Verbindung der Formel I 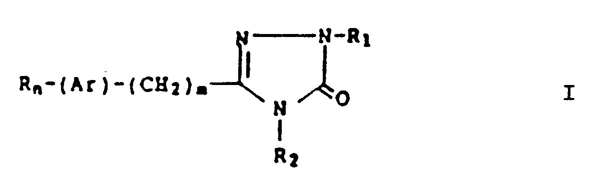 in der Rₙ-(Ar)-(CH₂)ₘ- eine 4-Chlorphenylgruppe bedeutet, R₁ eine Methylgruppe ist, und R₂ eine Ethylgruppe ist.

20. Verbindung der Formel I 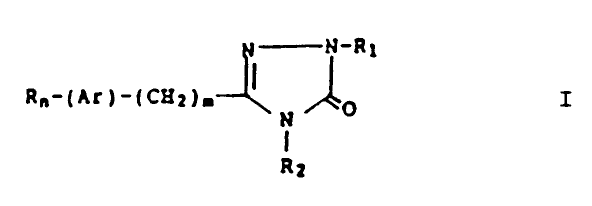 in der Rₙ-(Ar)-(CH₂)ₘ- eine 2-Chlorphenylgruppe bedeutet, R₁ ein Wasserstoffatom ist, und R₂ eine Methylgruppe ist.

21. Arzneimittelformulierung mit einer Verbindung nach einem der Ansprüche 11 bis 20 im Gemisch mit einem pharmazeutisch verträglichen Träger.

22. Verfahren zur Herstellung einer Verbindung der Formel 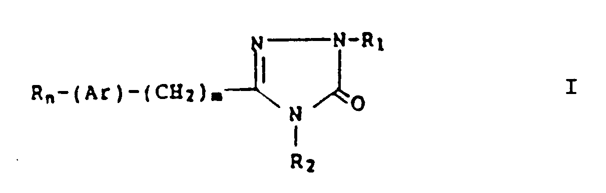 in der
Rₙ-(Ar)-(CH₂)ₘ- eine 4-Chlorphenylgruppe bedeutet, R₁ eine Methylgruppe bedeutet, und R₂ eine Ethylgruppe bedeutet, das die Umsetzung eines Semicarbazids der Formel IV
Rₙ-(Ar)-(CH₂)ₘCONHNHCONHR₂
in der Rₙ-(Ar)-(CH₂)ₘ- wie vorstehend definiert ist, mit einer Base bei etwa 50°C-120°C und die Umwandlung der erhaltenen Zwischenverbindung, in der R₁ ein Wasserstoffatom bedeutet, in die Verbindung der Formel I umfaßt, wobei sie mit einer Verbindung der Formel R₁X umgesetzt wird, in der X eine gute Austrittsgruppe ist, und R₁ eine Methylgruppe ist.

23. Verfahren zur Herstellung einer Verbindung der Formel 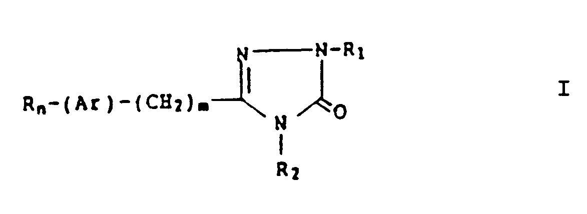 in der
Rₙ-(Ar)-(CH₂)ₘ- eine 2-Chlorphenylgruppe bedeutet, R₁ ein Wasserstoffatom bedeutet, und R₂ eine Methylgruppe bedeutet, das die Umsetzung eines Semicarbazids der Formel IV
Rₙ-(Ar)-(CH₂)ₘCONHNHCONHR₂
in der Rₙ-(Ar)-(CH₂)ₘ- wie vorstehend definiert ist, mit einer Base bei etwa 50°C-120°C umfaßt.

## Patentansprüche (Patentansprüche für folgende(n) Vertragsstaat(en): ES)

1. Verfahren zur Herstellung einer Verbindung der Formel 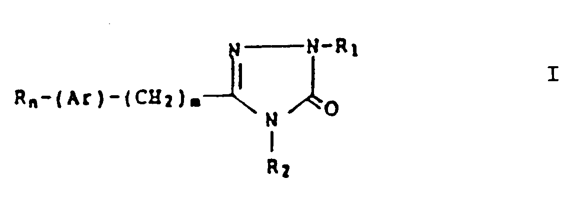 in der
Rₙ-(Ar)-(CH₂)ₘ- eine 4-Chlorphenylgruppe bedeutet, R₁ eine Methylgruppe bedeutet, und R₂ eine Ethylgruppe bedeutet, das die Umsetzung eines Semicarbazids der Formel IV
Rₙ-(Ar)-(CH₂)ₘCONHNHCONHR₂
in der Rₙ-(Ar)-(CH₂)ₘ- wie vorstehend definiert ist, mit einer Base bei etwa 50°C-120°C und die Umwandlung der erhaltenen Zwischenverbindung, in der R₁ ein Wasserstoffatom bedeutet, in die Verbindung der Formel I umfaßt, wobei sie mit einer Verbindung der Formel R₁X umgesetzt wird, in der X eine gute Austrittsgruppe ist, und R₁ eine Methylgruppe ist.

2. Verfahren zur Herstellung einer Verbindung der Formel 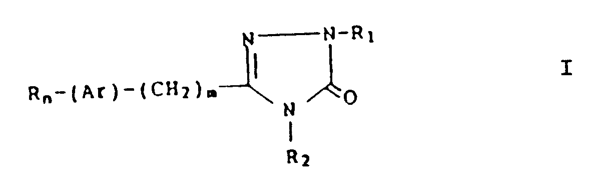 in der
Rₙ-(Ar)-(CH₂)ₘ- eine 2-Chlorphenylgruppe bedeutet, R₁ ein Wasserstoffatom bedeutet, und R₂ eine Methylgruppe bedeutet, das die Umsetzung eines Semicarbazids der Formel IV
Rₙ-(Ar)(CH₂)ₘCONHNHCONHR₂
in der Rₙ-(Ar)-(CH₂)ₘ- wie vorstehend definiert ist, mit einer Base bei etwa 50°C-120°C umfaßt.

## Revendications (Revendications pour l'(les) Etat(s) contractant(s) suivant(s): AT, BE, CH, DE, FR, GB, GR, IT, LI, LU, NL, SE)

1. Utilisation d'un composé de formule : 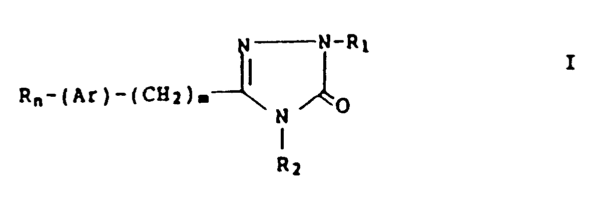 dans laquelle
Ar représente un groupe phényle, naphtyle ou une portion hérérocyclique aromatique choisie parmi les suivantes :
2-, 3- ou 4-pyridyle, 2- ou 3-furyle, 2- ou 3-thiényle, 2- ou 3-pyrrolyle, N-(C₁₋₆ alkyl)-pyrrolyle, 6-isoquinolyle, 6-quinolyle et 3-quinolyle,
R₁ est l'hydrogène ou C₁₋₆ alkyle,
R₂ est C₁₋₆ alkyle,
R est C₁₋₆ alkyle, C₁₋₆ alcoxy, hydroxy, halogéno ou trifluorométhyle, et
n est 0, 1 ou 2, ou
Rₙ-(Ar) est méthylènedioxyphényle, et
m est 0, 1 ou 2,
et des sels pharmaceutiquement acceptables des composés dans lesquels Ar est une portion hérérocyclique contenant de l'azote, pour la préparation d'un médicament pour le traitement des désordres de crises.

2. Utilisation d'un composé selon la revendication 1, selon laquelle Ar dans ledit composé représente un groupe phényle.

3. Utilisation d'un composé selon la revendication 1 ou 2, selon laquelle m dans ledit composé représente 0.

4. Utilisation d'un composé selon la revendication 1, 2 ou 3, selon laquelle R₁ dans ledit composé représente un groupe C₁₋₆ alkyle.

5. Utilisation d'un composé selon la revendication 1, 2 ou 3, selon laquelle R₁ dans ledit composé représente un groupe méthyle ou éthyle.

6. Utilisation d'un composé selon la revendication 1, selon laquelle Ar dans ledit composé est un groupe phényle, m est égal à 0 et R est un groupe halogéno.

7. Utilisation d'un composé selon la revendication 6, selon laquelle R dans ledit composé est un groupe chloro.

8. Utilisation d'un composé selon la revendication 1, selon laquelle Rₙ-(Ar)-(CH₂)ₘ-dans ledit composé représente un groupe 4-chlorophényle, R₁ est un groupe méthyle et R₂ est un groupe éthyle.

9. Utilisation d'un composé selon la revendication 1, selon laquelle Rₙ-(Ar)-(CH₂)ₘ-dans ledit composé représente un groupe 4-chlorophényle, R₁ est l'hydrogène et R₂ est un groupe éthyle.

10. Utilisation d'un composé selon la revendication 1, selon laquelle Rₙ-(Ar)-(CH₂)ₘ-dans ledit composé représente un groupe 2-chlorophényle, R₁ est l'hydrogène et R₂ est un groupe méthyle.

11. Un composé de formule 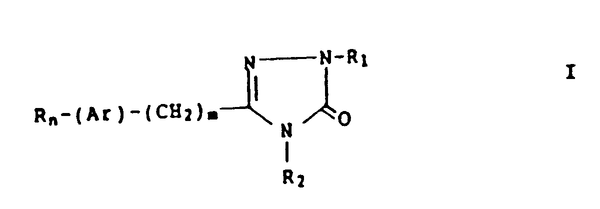 dans laquelle
Ar représente un groupe phényle, naphtyle ou une portion hérérocyclique aromatique choisie parmi les suivantes :
2-, 3- ou 4-pyridyle, 2- ou 3-furyle, 2- ou 3-thiényle, 2- ou 3-pyrrolyle, N-(C₁₋₆ alkyl)-pyrrolyle, 6-isoquinolyle, 6-quinolyle et 3-quinolyle,
R₁ est l'hydrogène ou C₁₋₆ alkyle,
R₂ est C₁₋₆ alkyle,
R est C₁₋₆ alkyle, C₁₋₆ alcoxy, hydroxy, halogéno ou trifluorométhyle, et
n est 1 ou 2, ou
Rₙ-(Ar) est méthylènedioxyphényle, et
m est 0, 1 ou 2,
à la condition que lorsque Rₙ-(Ar)-(CH₂)ₘ-représente un groupe 4-chlorophényle et R₂ représente un groupe éthyle, R₁ ne peut pas représenter l'hydrogène, et lorsque Rₙ-(Ar)-(CH₂)ₘ-représente un groupe phényle et R₁ représente un groupe méthyle, R₂ ne peut pas représenter un groupe méthyle ou éthyle, et les sels pharmaceutiquement acceptables des composés dans lesquels Ar est une portion hétérocyclique contenant de l'azote à utiliser comme médicament.

12. Un composé selon la revendication 11, selon laquelle Ar est un groupe phényle.

13. Un composé selon la revendication 11 ou 12, selon laquelle m est égal à 0.

14. Un composé selon la revendication 11, 12 ou 13, selon laquelle R₂ est un groupe méthyle ou éthyle.

15. Un composé selon la revendication 11, selon laquelle Ar est un groupe phényle, m est égal à 0 et R est un groupe halogéno.

16. Un composé selon la revendication 15, selon laquelle R est un groupe chloro.

17. Un composé selon la revendication 11, selon laquelle Rₙ-(Ar)-(CH₂)ₘ-représente un groupe 2-chlorophényle, R₁ est l'hydrogène et R₂ est un groupe méthyle.

18. Un composé selon la revendication 11, selon laquelle Rₙ-(Ar)-(CH₂)ₘ-représente un groupe 4-chlorophényle, R₁ est un groupe méthyle et R₂ est un groupe éthyle.

19. Un composé de formule I 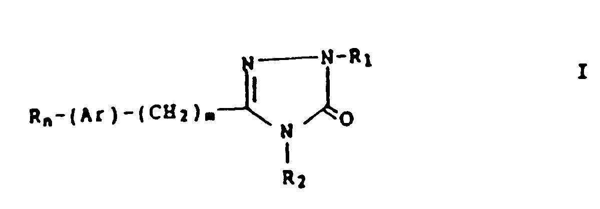 dans laquelle Rₙ-(Ar)-(CH₂)ₘ-représente un groupe 4-chlorophényle, R₁ est un groupe méthyle et R₂ est un groupe éthyle.

20. Un composé de formule I 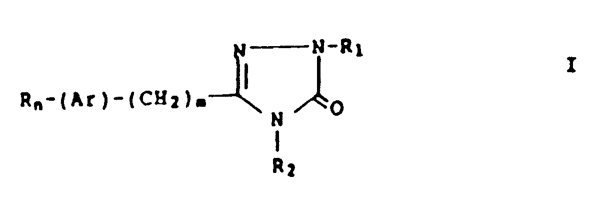 dans laquelle Rₙ-(Ar)-(CH₂)ₘ-représente un groupe 2-chlorophényle, R₁ est l'hydrogène et R₂ est un groupe méthyle.

21. Une formulation pharmaceutique contenant un composé selon l'une quelconque des revendications 11 à 20 en mélange avec un support pharmaceutique acceptable.

22. Un procédé de préparation d'un composé de formule 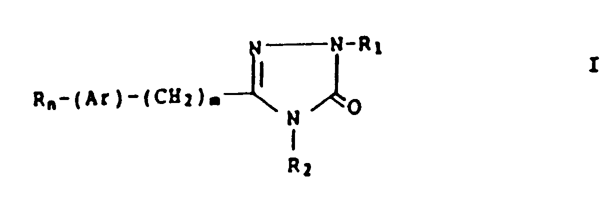 dans laquelle Rₙ-(Ar)-(CH₂)ₘ- représente un groupe 4-chlorophényle, R₁ représente un groupe méthyle et R₂ représente un groupe éthyle, qui comprend la réaction d'un semicarbazide de formule IV
Rₙ-(Ar)-(CH₂)ₘCONHNHCONHR₂
dans laquelle Rₙ-(Ar)-(CH₂)ₘ-est comme défini précédemment, avec une base à environ 50°C-120°C; et la transformation du produit intermédiaire obtenu dans lequel R₁ représente l'hydrogène en composé de formule I par réaction avec un composé de formule R₁X dans laquelle X est un bon groupe séparable et R₁ est un groupe méthyle.

23. Un procédé pour la préparation d'un composé de formule 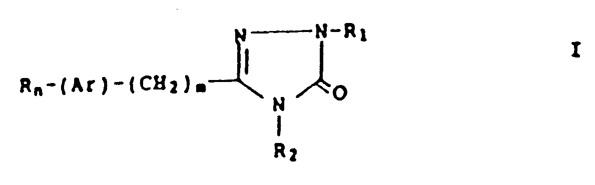 dans laquelle Rₙ-(Ar)-(CH₂)ₘ- représente un groupe 2-chlorophényle, R₁ représente l'hydrogène et R₂ représente un groupe méthyle qui comprend la réaction d'un semicarbazide de formule IV
Rₙ-(Ar)-(CH₂)ₘCONHNHCONHR₂
dans laquelle Rₙ-(Ar)-(CH₂)ₘ-est comme défini ci-dessus, avec une base à environ 50°C-120°C.

## Revendications (Revendications pour l'(les) Etat(s) contractant(s) suivant(s): ES)

1. Un procédé de préparation d'un composé de formule 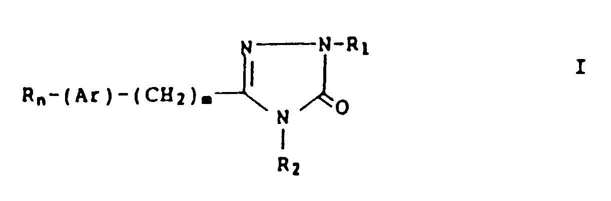 dans laquelle Rₙ-(Ar)-(CH₂)ₘ- représente un groupe 4-chlorophényle, R₁ représente un groupe méthyle et R₂ représente un groupe éthyle, qui comprend la réaction d'un semicarbazide de formule IV
Rₙ-(Ar)-(CH₂)ₘCONHNHCONHR₂
dans laquelle Rₙ-(Ar)-(CH₂)ₘ-est comme défini précédemment, avec une base à environ 50°C-120°C ; et la transformation de l'intermédiaire obtenu dans lequel R₁ représente l'hydrogène en composé de formule I par réaction avec un composé de formule R₁X dans lequel X est un bon groupe séparable et R₁ est un groupe méthyle.

2. Un procédé de préparation d'un composé de formule 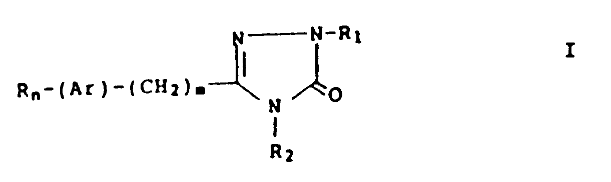 dans laquelle Rₙ-(Ar)-(CH₂)ₘ- représente un groupe 2-chlorophényle, R₁ représente l'hydrogène et R₂ représente un groupe méthyle qui comprend la réaction d'un semicarbazide de formule IV
Rₙ-(Ar)-(CH₂)ₘCONHNHCONHR₂
dans laquelle Rₙ-(Ar)-(CH₂)ₘ-est comme défini précédemment, avec une base à environ 50°C-120°C.
